# EUROPEAN PATENT APPLICATION

(11) **EP 4 361 263 A1**
(43) Date of publication of application: **01.05.2024**
(21) Application number: 22848281.6
(22) Date of filing: 14.07.2022
(51) Int. Cl.: C12N 13/00, C12N 5/00, C12N 5/09

(54) **BIOLOGICAL ASSEMBLY METHOD FOR FARADAY WAVE MULTI-WAVELENGTH SYNTHESIS AND APPLICATION**

(30) Priority: 30.07.2021 CN 202110872667
(71) Applicant: Shenzhen Convergence Bio-Manufacturing Co., Ltd, Shenzhen, Guangdong 518110 (CN)
(72) Inventor: CHEN, Pu, Shenzhen, Guangdong 518110 (CN); GU, Longjun, Shenzhen, Guangdong 518110 (CN); ZHENG, Lixin, Shenzhen, Guangdong 518110 (CN); WANG, Heng, Shenzhen, Guangdong 518110 (CN); ZHOU, Jinsheng, Shenzhen, Guangdong 518110 (CN)
(74) Representative: Kehl, Ascherl, Liebhoff & Ettmayr Patentanwälte Partnerschaft mbB
(86) International application number: PCT/CN2022/105626
(87) International publication number: WO 2023/005676

(57) **Abstract**

Provided are a biological assembly method for Faraday wave multi-wavelength synthesis and an application. The present method has advantages that the system is easy to bulid, manipulation is simple, patterns are dynamically adjustable, biocompatibility is good, etc. The present method is different from a cell manipulation principle under a single wavelength condition in an existing acoustic biological assembly method; sine or cosine signals having different wavelengths are synthesized, so that a single-wavelength assembly mode in a conventional Faraday wave frequency domain is improved into a multi-wavelength assembly mode, complex and arbitrary pattern arrangement of liquid-bottom multi-scale cells is achieved, and thus the method is more suitable for the requirements for complex arrangement of cells in tissue engineering and biological manufacturing, and has huge application prospects and commercial value.

## Description

### TECHNICAL FIELD

The present disclosure relates to the technical field of tissue engineering and biological manufacturing under biomedical engineering, and particularly relates to a biological assembly method and use for a Faraday wave multi-wavelength synthesis.

### BACKGROUND

In human tissues and organs, cells are interconnected through self-organization to form a highly ordered cell structure. The arrangement mode of cells in an in-vitro reduction structure and the geometrical shape of structure not only play a key role in simulating morphologies of in-vivo tissues and organs at a macroscopic scale, but also play an important role in regulating the microenvironment of cells.

In recent years, biological manufacturing has shown great potential in constructing cellular microstructures that highly restore the complexity and functionality of human tissues and organs. Biological manufacturing is to construct living cells, biological materials and stimulating factors into a biological product with specific structures and functions using biological printing or biological assembly, as well as subsequent tissue maturation processes. Among them, biological assembly is considered an important construction strategy in biological manufacturing, with the advantage that compared to biological printing, biological assembly can have better bio-compatibility and higher efficiency in constructing cell structures. So far, a series of biological assembly technologies are utilizing the interaction between external physical fields and cells to manipulate the arrangement of cells in space. Such physical fields comprise gravity fields, magnetic fields, and electric fields, etc. However, the cell structures constructed by biological assembly techniques based on the above principles often only have simple geometric shapes such as spherical, bar shaped, and circular, which are difficult to meet the needs of constructing multi-scale, complex and arbitrary cell structures.

Compared with other biological assembly technologies, acoustic biological assembly has the unique advantage of a dynamically adjustable sound field, so that it can provide better cell structure diversity. Among them, acoustic holography technology has been used to construct complex and arbitrary cell structures. However, this technology has the problems of being a complex system, complicated operation process, and incapability of adjusting the complex cell structure in real time. Compared to this technology, Faraday wave biological assembly technology has advantages such as easy system construction, simple operation, dynamically adjustable patterns, and good biocompatibility, etc. and has broad application prospects in the field of biological tissue engineering. However, the existing Faraday wave biological assembly technology only performs single-wavelength cell assembly in its frequency domain, and a limited cell structure in a single scale capable of being constructed fails to meet the requirements of constructing the multi-scale, complex and arbitrary cell structures.

Therefore, providing a biological assembly method that can meet the needs of achieving the complex and arbitrary arrangement of multi-scale cells in biological tissue engineering research has important application prospects and also technical promotion and application value.

### SUMMARY

The problem to be solved by the present disclosure is to provide a biological assembly method for multi-wavelength synthesis of a Faraday wave and an application thereof aiming at shortcomings in an existing biological assembly technology. The present disclosure is intended to excite and form the Faraday wave obtained by multi-wavelength synthesis by synthesizing a plurality of sine or cosine signals with different wavelengths, a single-wavelength assembly mode in a traditional Faraday wave frequency domain is expanded into a multi-wavelength assembly mode, and complex and arbitrary pattern arrangement of liquid-bottom multi-scale cells is realized, so that the biological assembly of the Faraday wave has higher technical popularization and application values.

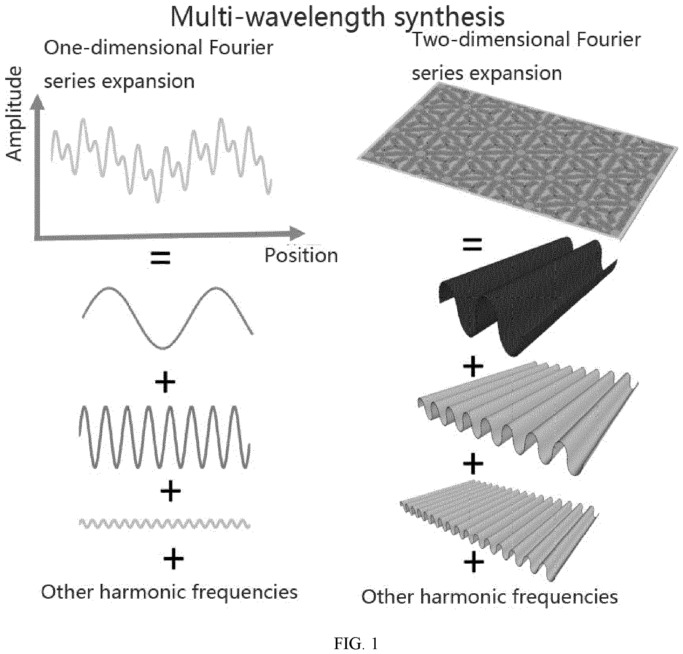

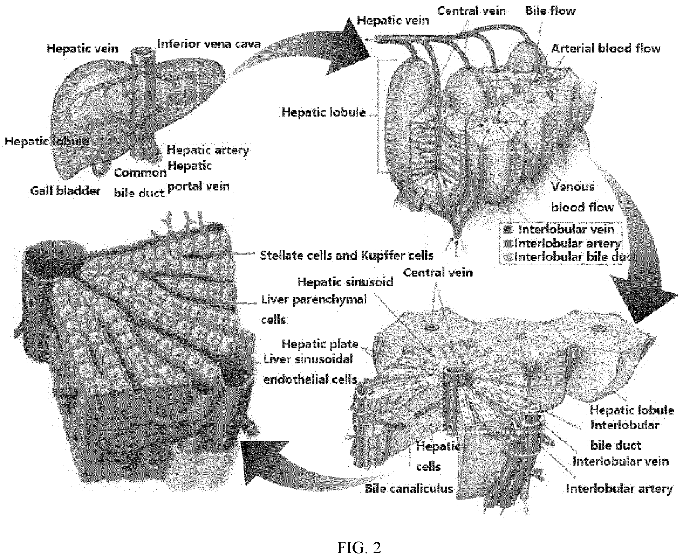

In order to achieve the above objective, **a working principle of the present disclosure is as follows:**
An acoustic biological assembly theory of multi-wavelength synthesis is established. In an acoustic field of Faraday waves, the liquid-bottom cells are subjected to the combined actions of acoustic pressure, buoyancy and gravity, wherein the acoustic pressure plays a decisive role in a cell's equilibrium position, and the cells are finally equilibrated to a position with the lowest potential energy in an acoustic pressure field, which is namely a potential well position. Any complex acoustic pressure fields may be simplified into superposition of a series of sine or cosine acoustic pressure fields by a Fourier series. Therefore, a plurality of sine or cosine signals with different wavelengths may be synthesized into a complex waveform, so as to meet the requirements of complex and arbitrary arrangement of multi-scale cells. A multi-scale complex structure of multi-wavelength synthesis is shown in FIG. 1. For example, the liver is an important organ with a unique multi-scale structure, with an adult liver being composed of 0.5 to 1 million hexagonal hepatic lobules. A periodic hepatic lobule structural unit and a multi-scale micro-physiological structure of a liver tissue are shown in FIG. 2. Therefore, a complex liver tissue structure may be simplified into a liver lobule structure arranged repeatedly in space. Complex acoustic pressure field distribution is realized in space by multi-wavelength synthesis, thus realizing the construction of a multi-scale liver cell structure. In addition, there are a large number of tissues and organs in the human body that contain specific multi-scale structural units, for example, pulmonary alveoli are mainly hemispherical vesicles composed of monolayer epithelial cells; and renal tubules are mainly hollow tubular structures formed by self-organization of renal tubular epithelial cells. Physiological structures of these specific tissues and organs may all be constructed in vitro by assembly for the multi-wavelength synthesis of the Faraday wave.

In order to achieve the above objective, **a technical solution of the present disclosure is as follows:**
In a first aspect, the present disclosure provides a cell assembly method for multi-wavelength synthesis of Faraday waves, wherein any complex periodic pattern is capable of being simplified into superposition of a series of sine or cosine waves by a Fourier series, with a plurality of sine or cosine signals with different wavelengths being synthesized in the method, thus exciting and forming the Faraday wave obtained by multi-wavelength synthesis, and finally, complex and arbitrary arrangement of multi-scale cells is realized; while the node is a node position of a Faraday standing wave, the anti-node is an anti-node position of the Faraday standing wave, and the method comprises the following steps:
S1: Synthesizing the sine or cosine signals with different wavelengths in a waveform creating and editing tool software according to a biological assembly pattern to be constructed, and inputting a synthesized multi-wavelength signal file into an arbitrary waveform/function signal generator;
S2: Opening the multi-wavelength signal file synthesized in the S1 in the arbitrary waveform/function signal generator, outputting a corresponding electric signal through the arbitrary waveform/function signal generator, transferring the electric signal to a power amplifier for power amplification, transferring the amplified fidelity electric signal to a vibration exciter to generate stable and periodic vibration, and connecting an assembly chamber with the vibration exciter for horizontal calibration; and
S3: Evenly adding a suspension of cell-containing assembly units to be assembled into the assembly chamber, and carrying out assembly for the multi-wavelength synthesis of the Faraday wave after the cell-containing assembly unit sediments to a bottom portion of the assembly chamber.

Specific steps are as follows.

In S1, according to a cell pattern arranged by target assembly, the synthesis of Faraday waves with different wavelengths is realized in the waveform creating and editing tool software, and the frequency is selected in range from 1 Hz to 1,000 Hz. For example, for synthesis of a dual-wavelength Faraday wave, a written sine signal function is *y* = *A*₁ * sin(*f*₁ * 2 * *π* * *x*) + *A*₂ * sin(*f*₂ * 2 * *π* * *x*); and a written cosine signal function is *y* = *A*₁ * cos(*f*₁ * 2 * *π* * *x*) + *A*₂ * cos(*f*₂ * 2 * *π* * *x*). *f*₁ is a low driving frequency, *f*₂ is a high driving frequency, and the ratio of a corresponding amplitude *A*₁ of the sine or cosine signal function of the low driving frequency to a corresponding amplitude *A*₂ of the sine or cosine signal function of the high driving frequency ranges from 1: 1 to 1: 5. For synthesis of a multi-wavelength Faraday wave, a written sine signal function is *y* = *A*₁ * sin(*f*₁ * 2 * *π* * *x*) + *A*₂ * sin(*f*₂ * 2 * *π* * *x*) + *A*₃ * sin(*f*₃ * 2 * *π* * *x*) + *A*₄ * sin(*f*₄ * 2 * *π* * *x*) + *A*₅ * sin(*f*₅ * 2 * *π* * *x*) + ···; and a written cosine signal function is *y* = *A*₁ * cos(*f*₁ * 2 * *π* * *x*) + *A*₂ * cos(*f*₂ * 2 * *π* * *x*) + *A*₃ * cos(*f*₃ * 2 * *π* * *x*) + *A*₄ * cos(*f*₄ * 2 * *π* * *x*) + *A*₅ * cos(*f*₅ * 2 * *π* * *x*) + ···. The frequencies satisfy that *f*₁ *< f*₂ < *f*₃ < *f*₄ *< f*₅ ; the amplitudes satisfy that *A*₁ ≤ *A*₂ ≤ *A*₃ ≤ *A*₄ ≤ *A*₅, and a ratio of a lower amplitude to a higher amplitude ranges from 1: 1 to 1: 5. The synthesized multi-wavelength signal file is input into the arbitrary waveform/function signal generator.

In S2, the synthesized multi-wavelength signal file is opened, the specific electric signal is output through the arbitrary waveform/function signal generator, the electric signal is transferred to the power amplifier for power amplification, and the amplified fidelity electric signal is transferred to the vibration exciter to generate stable and periodic vibration. The assembly chamber is connected with the vibration exciter for horizontal calibration. A shape of the assembly chamber may be a circle, a square, a rectangle, a triangle, a trapezoid, a diamond, a hexagon and an octagon; and the assembly chamber in each shape has a circumscribed circle diameter of 0.5 cm to 20 cm; and a height of 0.2 mm to 10 mm.

In the S3, in the cell-containing assembly unit used for assembly, a selected cell is one or a mixture of several of either an embryonic stem cell, an induced pluripotent stem cell, a cancer stem cell, or a mesenchymal stem cell; or, the selected cell is any one of the above or a mixture of several of primary cells, progenitor cells, precursor cells, or diseased cells of tissues and organs comprising brain, liver, kidney, pancreas, blood vessel, heart, skin, bone marrow, bone, cartilage and muscle. The cell-containing assembly unit is one or a mixture of several of either a single cell, a micro-tissue block, an organoid, a cell microsphere, a cell-containing hydrogel microsphere and a cell-containing carrier particle, with a diameter of 2 µm to 5,000 µm. The number of the cell-containing assembly units range from 2 to 10¹². The cell-containing assembly units are evenly dispersed in a phosphate buffered solution, a cell culture medium, a natural hydrogel, a synthetic hydrogel, or a mixed hydrogel for later use, with a buoyant density of 1 g/cm³ to 10 g/cm³. The suspension of the cell-containing assembly unit to be assembled is evenly added into the assembly chamber, and assembly for the multi-wavelength synthesis of the Faraday wave is carried out after the cell-containing assembly unit sediments to the bottom portion of the assembly chamber.

In a second aspect, the present disclosure provides an application of the cell assembly method for the multi-wavelength synthesis of the Faraday wave described above, wherein the method is applied to construction of artificial tissues and organs by using a biological assembly unit containing living cells, and the constructed artificial tissues and organs are capable of being used in cellular artificial meat, a drug test model, and a clinical tissue and organ repair product.

**The present disclosure has the advantages and the generated beneficial effects as follows.**

The cell assembly method for the multi-wavelength synthesis of the Faraday waves according to the present disclosure has the advantages of easy establishment of a system, simple manipulation, a dynamically adjustable pattern, good biocompatibility, and the like. The present disclosure is different from other cell manipulation techniques in principle under a single-wavelength condition in an existing acoustic biological assembly method, in which sine or cosine signals with different wavelengths are synthesized, thus exciting and forming the Faraday wave obtained by multi-wavelength synthesis, a single-wavelength assembly mode in a traditional Faraday wave frequency domain is expanded into a multi-wavelength assembly mode, and complex and arbitrary pattern arrangements of liquid-bottom multi-scale cells is realized, so that the present disclosure is more suitable for the requirements of complex arrangement of cells in tissue engineering and biological manufacturing, and has huge application prospects and commercial conversion potential.

### DESCRIPTION OF THE DRAWINGS

FIG. 1 is a schematic diagram of a multi-scale complex structure of multi-wavelength synthesis.
FIG. 2 is a schematic diagram of the periodic structural units and multi-scale micro-physiological structures of liver tissue.
FIG. 3 is a schematic diagram of single-wavelength, dual-wavelength and three-wavelength polystyrene microsphere patterns obtained by liquid-bottom assembly in Example 1 of the present disclosure.
FIG. 4 is a schematic diagram of the single-wavelength and dual-wavelength HepG2 cell microsphere patterns obtained by liquid-bottom assembly in Example 2 of the present disclosure.

### DETAILED DESCRIPTION

The following will explain the technical solution of the present disclosure in conjunction with examples. Those skilled in the art will understand that the following examples are only intended to illustrate the present disclosure and should not be considered to limit the scope of the present disclosure. Specific technologies or conditions not specified in the examples should follow the technologies or conditions described in the literature in the art or be carried out in accordance with the product specification.

### Example 1: Assembly of Faraday wave multi-wavelength synthesis for the arrangement of polystyrene microspheres

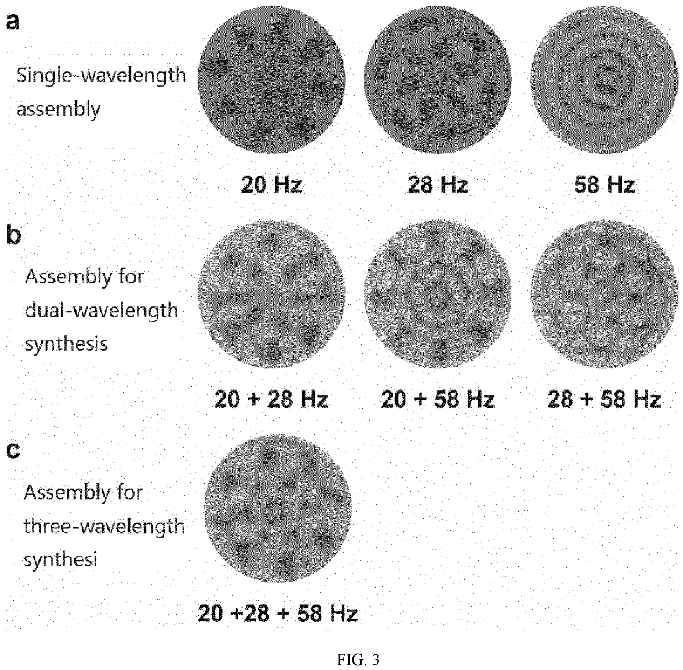

Polystyrene microspheres were added into 0.01 M phosphate buffer solution containing 0.05% Tween 20 and fully dispersed. The polystyrene microspheres were red in color, and had a diameter of 100 µm, a density of 1.05 g/cm³, and physical properties similar to that of cell-containing assembly units, and were at a concentration of 30 mg/mL. In this example, the polystyrene microspheres were used to simulate the cell-containing assembly units.
1. The arrangement of polystyrene microspheres under single-wavelength conditions, steps are as follows:
   After setting the waveform as a continuous sine wave, the driving frequencies were set to be 20 Hz, 28 Hz and 58 Hz, and the signal amplitude was 60 mVpp to 100 mVpp. A specific electric signal was output by adjusting a waveform/function signal generator (Tecktronix, AFG3052C), the electric signal was transferred to a power amplifier (DAYTONAUDIO, DTA-120) through a BNC bus-to-RCA common line for power amplification. The amplified fidelity electric signal was transferred to a vibration exciter (Wuxi Shiao Technology Co., Ltd., SA-JZ005T) through an RCA common converted four-core socket to generate stable and periodic vibrations. After the circular assembly chamber (Φ=3 cm) was rigidly connected to the vibration exciter through M5×30, 360° horizontal calibration was carried out by a circular spirit level. Finally, the suspension of polystyrene microspheres to be assembled was evenly added into the circular assembly chamber, and Faraday wave assembly was carried out after the polystyrene microspheres settled to the bottom of the assembly chamber. The pattern obtained by assembling polystyrene microspheres under single wavelength conditions was shown in **FIG. 3 (a)**, which can only form relatively simple divergent, petal-shaped and concentric circular patterns.
2. The arrangement of polystyrene microspheres under dual-wavelength synthesis condition, steps are as follows:
   The synthesis of two sine signals with different wavelengths was realized by using "ArbExpress" software. The wave function of the synthesized sine signal synthesized at the corresponding wavelengths of driving frequencies 20 Hz and 28 Hz was *y* = 1.5 × sin(20 × 2 × *π* × *x*) + sin(28 × 2 × *π* × *x*). The wave function of the synthesized sine signal at the corresponding wavelengths of driving frequencies 20 Hz and 58 Hz was *y* = 1.2 × sin(20 × 2 × *π* × *x*) + sin(58 × 2 × *π* × *x*). The wave function of the synthesized sine signal at the corresponding wavelengths of driving frequencies 28 Hz and 28 Hz was *y* = sin(20 × 2 × *π* × *x*) + sin(28 × 2 × *π* × *x*). The synthesized dual-wavelength sine signal was saved as a "TFW" format file, and imported into a waveform/function signal generator (Tecktronix, AFG3052C).
   After setting the waveform to the desired waveform, the synthesized dual-wavelength sine signal files were opened in sequence, and the signal amplitude was 140 mVpp to 160 mVpp. By outputting a specific electric signal through a waveform/function signal generator, the electric signal was transferred to the power amplifier (DAYTONAUDIO, DTA-120) through a BNC bus-to-RCA common line for power amplification. The amplified fidelity electric signal was transferred to a vibration exciter (Wuxi Shiao Technology Co., Ltd., SA-JZ005T) through an RCA common converted four-core socket to generate stable and periodic vibrations. After the circular assembly chamber (Φ=3 cm) was rigidly connected to the vibration exciter through M5×30, 360° horizontal calibration was carried out by a circular spirit level. Finally, the suspension of polystyrene microspheres to be assembled was evenly added into the circular assembly chamber, and Faraday wave assembly was carried out after the polystyrene microspheres settled to the bottom of the assembly chamber. The pattern obtained by assembling polystyrene microspheres under dual-wavelength conditions is shown in **FIG. 3 (b)**, which achieves the superposition of corresponding patterns under the single-wavelength conditions, resulting in a pattern of divergent, petal-shaped and concentric circular patterns overlapping each other.
3. The arrangement of polystyrene microspheres under three-wavelength synthesis conditions, steps are as follows:
   The synthesis of three sine signals with different wavelengths was realized by using "ArbExpress" software. The wave function of the synthesized sine signal at the corresponding wavelengths of driving frequencies 20 Hz, 28 Hz and 58 Hz was *y* = 1.4 × sin(20 × 2 × *π* × *x*) + sin(28 × 2 × *π* × *x*) + sin(58 × 2 × *π* × *x*). The synthesized three-wavelength sine signal was saved as a "TFW" format file, and imported into a waveform/function signal generator (Tecktronix, AFG3052C).

After setting the waveform to the desired waveform, the synthesized three-wavelength sine signal file was opened, and the signal amplitude was 200 mVpp. By outputting a specific electric signal through a waveform/function signal generator, the electric signal was transferred to the power amplifier (DAYTONAUDIO, DTA-120) through a BNC bus-to-RCA common line for power amplification. The amplified fidelity electric signal was transferred to a vibration exciter (Wuxi Shiao Technology Co., Ltd., SA-JZ005T) through an RCA common converted four-core socket to generate stable and periodic vibration. After the circular assembly chamber (Φ=3cm) was rigidly connected to the vibration exciter through M5×30, 360° horizontal calibration was carried out by a circular spirit level. Finally, the suspension of polystyrene microspheres to be assembled was evenly added into the circular assembly chamber, and Faraday wave assembly was carried out after the polystyrene microspheres settled to the bottom of the assembly chamber. The pattern obtained by assembling polystyrene microspheres under three-wavelength conditions is shown in **FIG. 3 (c)****,** which further achieves the complex and arbitrary pattern arrangement of the polystyrene microspheres under multi-scale conditions, and the assembly pattern had characteristics of divergent, petal-shaped and concentric circular patterns all at the same time.

### Example 2: Assembly of Faraday wave multi-wavelength synthesis for the arrangement of HepG2 cell microspheroids

The arrangement of the HepG2 cell microspheroids under single-wavelength condition, steps are as follows:
HepG2 cells were added into a six-well low-adhesion plate (Corning, 3471), with 2×10⁶ cells in each well, and added with 2 mL of DMEM complete culture medium. The six-well low-adhesion plate was placed on a flat shaker at a rotational speed of 75 rpm, and cultured for three days to form cell spheroids. HepG2 cell microspheroids in one well were collected and added with 0.01 M phosphate buffer solution for later use.

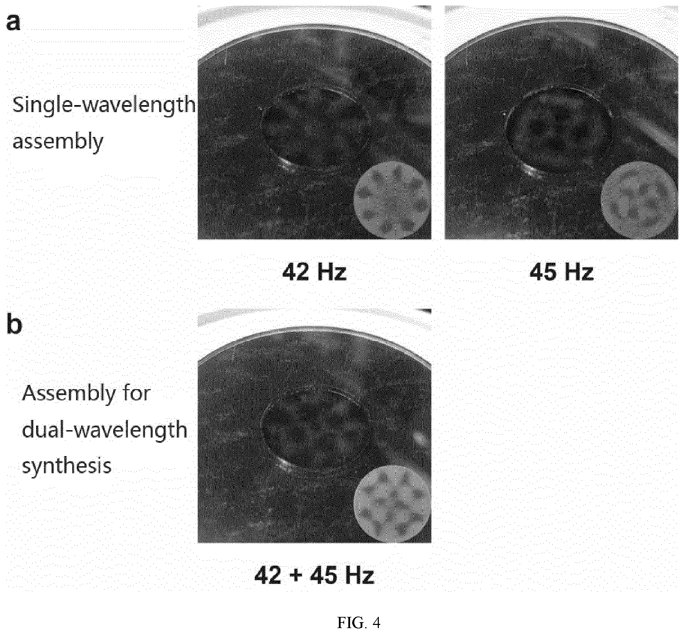

After setting the waveform as a continuous sine wave, the driving frequency was set to be 42 Hz and 45 Hz, and the signal amplitude was 60 mVpp to 80 mVpp. A specific electric signal was output by adjusting a waveform/function signal generator (Tecktronix, AFG3052C), the electric signal was transferred to a power amplifier (DAYTONAUDIO, DTA-120) through BNC bus-to-RCA common line for power amplification. The amplified fidelity electric signal was transferred to a vibration exciter (Wuxi Shiao Technology Co., Ltd., SA-JZ005T) through an RCA common converted four-core socket to generate stable and periodic vibration. After the black-background circular assembly chamber (Φ=2 cm) with black background was rigidly connected to the vibration exciter through M5×30, 360° horizontal calibration was carried out by a circular spirit level. Finally, the suspension of HepG2 cell microspheroids to be assembled was evenly added into the circular assembly chamber, and Faraday wave assembly was carried out after the HepG2 cell microspheroids settled to the bottom of the assembly chamber. The pattern obtained by assembling the HepG2 cell microspheroids under single wavelength conditions was shown in **FIG. 4 (a)****,** which can only form relatively simple divergent and four-leaf petal patterns.

The arrangement of the HepG2 cell microspheroids under dual-wavelength synthesis condition, steps as follows:
The synthesis of two sine signals with different wavelengths was realized by using "ArbExpress" software. The wave function of the synthesized sine signal at the corresponding wavelengths of driving frequencies 42 Hz and 45 Hz was *y* = 2.1 × sin(42 × 2 × *π* × *x*) + sin(45 × 2 × *π* × *x*). The synthesized dual-wavelength sine signal was saved as a "TFW" format file, and imported into any waveform/function signal generator (Tecktronix, AFG3052C).

After setting the waveform as any waveform, the synthesized dual-wavelength sine signal file was opened, and the signal amplitude was 140 mVpp to 160 mVpp. The assembly of the HepG2 cell microspheroids under the dual-wavelength condition was completed by aforementioned Faraday wave acoustic biological assembly system. The pattern obtained by assembling HepG2 cell microspheroids under the dual-wavelength condition was shown in **FIG. 4 (b)****,** which achieves the superposition of corresponding patterns under the single-wavelength conditions. Under the multi-scale condition, HepG2 cell microspheroids are arranged in complex and arbitrary patterns, with assembly patterns had characteristics of divergent and four-leaf petal patterns at the same time.

**In summary, the present disclosure innovatively synthesizes a plurality of sine or cosine signals of different wavelengths to excite and form the Faraday wave for multi-wavelength synthesis, achieving complex and arbitrary cell arrangement pattern under the multi-scale condition. The system in this method is easy to construct, simple to operate, dynamically adjustable in pattern, and has better biocompatibility. More importantly, this method differs from the cell manipulation principle of existing acoustic biological assembly methods under single-wavelength condition. By exciting the formation of Faraday waves for multi-wavelength synthesis, the single-wavelength assembly mode in the traditional Faraday wave frequency domain is expanded a multi-wavelength assembly mode, achieving multi-scale, complex and an arbitrary pattern of cells arrangement at the liquid bottom. It provides an innovative solution to the demand for multi-scale, complex and arbitrary cells arrangement in tissue engineering and biological manufacturing application.**

## Claims

1. A biological assembly method for Faraday wave multi-wavelength synthesis, wherein: according to any complex periodic pattern is capable of being simplified into superposition of a series of sine or cosine waves by a Fourier series, multiple sine or cosine signals of different wavelengths are synthesized in the method to excite and form Faraday waves for multi-wavelength synthesis, and ultimately achieving multi-scale, complex and arbitrary cells arrangement; and the method comprises the following steps of
S1: synthesizing the sine or cosine signals of different wavelengths in a waveform creating and editing tool software according to a biological assembly pattern to be constructed, and importing a synthesized multi-wavelength signal file into a waveform/function signal generator;
S2: opening the synthesized multi-wavelength signal file in S1 in a waveform/function signal generator, outputting a corresponding electric signal through the arbitrary waveform/function signal generator, transferring the electric signal to a power amplifier for power amplification, transferring the amplified fidelity electric signal to a vibration exciter to generate stable and periodic vibration, and connecting assembly chamber with the vibration exciter for horizontal calibration; and
S3: evenly adding a cell-containing assembly unit suspension to be assembled into the assembly chamber, and carrying out assembly for the Faraday wave multi-wavelength synthesis after the cell-containing assembly unit settled to bottom of the assembly chamber.

2. The biological assembly method for Faraday wave multi-wavelength synthesis according to claim 1, wherein:
in the step S1, synthesis of multiple sine or cosine signals, specifically are:
for synthesis of a dual-wavelength Faraday wave, a written sine signal function is *y* = *A*₁ × sin(*f*₁ × 2 × *π* × *x*) + *A*₂ × sin(*f*₂ × 2 × *π* × *x*), wherein *f*₁ is a low driving frequency, *f*₂ is a high driving frequency, *A*₁ is a corresponding amplitude of the sine signal function of the low driving frequency, and *A*₂ is a corresponding amplitude of the sine signal function of the high driving frequency; and a written cosine signal function is *y* = *A*₁ × cos(*f*₁ × 2 × *π* × *x)* + *A*₂ × cos(*f*₂ × 2 × *π* × *x*), wherein *f*₁ is a low driving frequency, *f*₂ is a high driving frequency, *A*₁ is a corresponding amplitude of the sine signal function of the low driving frequency, and *A*₂ is a corresponding amplitude of the sine signal function of the high driving frequency; and for Faraday wave multi-wavelength synthesis, a written sine signal function is *y* = *A*₁ × sin(*f*₁ × 2 × *π* × *x*) + *A*₂ × sin(*f*₂ × 2 × *π* × *x*) + *A*₃ × sin(*f*₃ × 2 × *π* × *x*) + *A*₄ × sin(*f*₄ × 2 × *π* × *x*) + *A*₅ × sin(*f*₅ × 2 × *π* × *x*) + ···; and a written cosine signal function is *y* = *A*₁ × cos(*f*₁ × 2 × *π* × *x*) + *A*₂ × cos(*f*₂ × 2 × *π* × *x*) + *A*₃ × cos(*f*₃ × 2 × *π* × *x*) + *A*₄ × cos(*f*₄ × 2 × *π* × *x*) + *A*₅ × cos(*f*₅ × 2 × *π* × *x*) + ···.

3. The biological assembly method for Faraday wave multi-wavelength synthesis according to claim 2, wherein: the driving frequency is selected from the range of 1 Hz to 1,000 Hz.

4. The biological assembly method for Faraday wave multi-wavelength synthesis according to claim 3, wherein: in the synthesis of dual-wavelength Faraday wave, the ratio of the corresponding amplitude *A*₁ of the sine or cosine signal function of the low driving frequency to the corresponding amplitude *A*₂ of the sine or cosine signal function of the high driving frequency ranges form 1: 1 to 1: 5; and in the synthesis of multi-wavelength Faraday wave, the driving frequencies *f*₁ *< f*₂ < *f*₃ < *f*₄ < *f*₅, the amplitudes *A*₁ ≤ *A*₂ ≤ *A*₃ ≤ *A*₄ ≤ *A*₅, and the ratio of a lower amplitude to a higher amplitude ranges from 1: 1 to 1: 5.

5. The biological assembly method for Faraday wave multi-wavelength synthesis according to any one of claims 1 to 4, wherein: in the step S2, a shape of the assembly chamber is any one of a circle, a square, a rectangle, a triangle, a trapezoid, a diamond, a hexagon or an octagon, and the assembly chamber in each shape has a circumscribed circle diameter of 0.5 cm to 20 cm; and a height of 0.2 mm to 10 mm.

6. The biological assembly method for Faraday wave multi-wavelength synthesis according to claim 1, wherein: in the step S3, the cell-containing assembly unit are any one or a mixture of several of a single cell, a micro-tissue block, an organoid, a cell microspheroid, a cell-containing hydrogel microsphere and a cell-containing carrier particle, with a diameter of 2 µm to 5,000 µm.

7. The biological assembly method for Faraday wave multi-wavelength synthesis according to claim 1, wherein: in the step S3, the number of the cell-containing assembly units is 2 to 10¹².

8. The biological assembly method for Faraday wave multi-wavelength synthesis according to claim 6 or 7, wherein: in the step S3, the cell is selected from the group consisting of any one or a mixture of several of an embryonic stem cell, an induced pluripotent stem cell, a cancer stem cell, and a mesenchymal stem cell; or, the cell is selected from the group consisting of any one or a mixture of several of primary cells, progenitor cells, precursor cells and diseased cells thereof of tissues and organs comprising brain, liver, kidney, pancreas, blood vessel, heart, skin, bone marrow, bone, cartilage and muscle.

9. The biological assembly method for Faraday wave multi-wavelength synthesis according to claim 8, wherein: in the step S3, the suspension system is any one of a phosphate buff solution, a cell culture medium, a natural hydrogel, a synthetic hydrogel or a mixed hydrogel.

10. The biological assembly method for Faraday wave multi-wavelength synthesis according to claim 1, wherein: the multi-scale, complex and arbitrary cells arrangement refers to cells arranged in a Faraday wave node and/or anti-node assembly pattern.

11. The biological assembly method for Faraday wave multi-wavelength synthesis according to claim 10, wherein: the node is a node position of a Faraday standing wave, and the anti-node is an anti-node position of the Faraday standing wave.

12. The biological assembly method for Faraday wave multi-wavelength synthesis according to claim 1, wherein: in the step S3, the cell-containing assembly unit is one or more assembly units containing different cell types.

13. The biological assembly method for Faraday wave multi-wavelength synthesis according to claim 1, wherein:
in the step S3, the cell-containing assembly unit comprises any one or a mixture of several of single cell, micro-tissue block, organoid, cell microspheroid, cell-containing hydrogel microsphere and cell-containing carrier particle, with a buoyant density of 1 g/cm³ to 10 g/cm³.

14. Use of the biological assembly method for Faraday wave multi-wavelength synthesis according to any one of claims 1 to 4 or 6 to 13, wherein: the method is applied to construction of artificial tissues and organs by using biological assembly unit containing living cells, and the artificial tissues and organs constructed are capable of being used in cellular artificial meat, a drug test model, a clinical tissue and an organ repair product.
